(19) 

**Europäisches Patentamt**
**European Patent Office**
**Office européen des brevets**

(11) **EP 4 014 906 A1**

(12) **EUROPEAN PATENT APPLICATION**
published in accordance with Art. 153(4) EPC

(43) Date of publication:
**22.06.2022 Bulletin 2022/25**

(21) Application number: **20851650.0**

(22) Date of filing: **06.08.2020**

(51) International Patent Classification (IPC):
***A61B 18/02*** *(2006.01)*

(86) International application number:
**PCT/CN2020/107401**

(87) International publication number:
**WO 2021/027682 (18.02.2021 Gazette 2021/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.08.2019 CN 201910748848**

(71) Applicant: **Synaptic Medical Technology (Beijing) Co., Ltd.**
**Beijing 101111 (CN)**

(72) Inventors:
• **GONG, Jie**
  **Beijing 101111 (CN)**
• **HAN, Boyang**
  **Beijing 101111 (CN)**

• **LIU, Yanbin**
  **Beijing 101111 (CN)**
• **FENG, Ji**
  **Beijing 101111 (CN)**
• **PENG, Bo**
  **Beijing 101111 (CN)**
• **LIU, Cuihu**
  **Beijing 101111 (CN)**
• **WEI, Honggang**
  **Beijing 101111 (CN)**
• **WANG, Xiaolong**
  **Beijing 101111 (CN)**

(74) Representative: **Ainscow, Georgina Frances**
**Reddie & Grose LLP**
**The White Chapel Building**
**10 Whitechapel High Street**
**London E1 8QS (GB)**

(54) **TEMPERATURE-CONTROLLABLE CRYOABLATION SYSTEM**

(57)    Provided is a temperature-controllable cryoablation system, comprising a catheter, a fluid-conveying unit and a control unit, wherein the catheter comprises a central cavity and a balloon located at the distal end of the catheter, and an input channel allowing a freezing fluid to be input into the balloon and an outflow channel allowing the freezing fluid to flow out of the balloon are provided in the central cavity; the fluid-conveying unit supplies the freezing fluid and discharges the freezing fluid; and the control unit controls the fluid-conveying unit so as to control, according to a target temperature value, the temperature of the balloon to be close to the target temperature value. When the cryoablation system works stably, the temperature of the balloon at the distal end of the catheter can be close to the target temperature value by controlling an input pressure of the freezing fluid, such that the operative risk is small, and a safer and more stable ablation mode is achieved when the same ablation depth is reached.

FIG. 1

**Description**

**TECHNICAL FIELD**

The present invention relates to a cryoablation system, and particularly to a balloon-freezing and temperature-controllable cryoablation system.

**BACKGROUND**

Currently, in the view of therapeutic risk of radiofrequency ablation to patients with arrhythmia, interventional cryoablation technology begins to be applied in clinical treatments. In cryoablation, liquefied gas, such as liquid nitrogen, is adopted as a freezing fluid source, and heat of tissue is taken away by absorption and evaporation of the liquid nitrogen, such that a temperature of a target site is reduced to a certain temperature, and cell tissue with electrophysiological abnormalities is damaged without a cold injury to the tissue, thereby eliminating an arrhythmia risk.

However, the state-of-art cryoablation system has certain limitation and is only able to operate with constant refrigeration power, and unable to meet different requirements of treatment on the lowest temperature in the atrial fibrillation cryoablation, thus requiring a more complete cryoablation system capable of controlling different freezing temperatures.

**SUMMARY**

In a cryoablation system according to a preferred embodiment of the present invention, by stably controlling a freezing fluid, an input amount of the freezing fluid may be controlled effectively, such that the freezing fluid may be effectively gasified in a balloon at the distal end of a catheter, thus achieving an optimal refrigeration effect of the balloon. When the cryoablation system works stably, a stable pressure value always exists in the balloon at the distal end of the catheter, and a temperature of the balloon may be close to a target temperature value by controlling an input pressure of the freezing fluid, such that an operative risk is small, and a safer and more stable ablation mode is achieved when the same ablation depth is reached.

According to an aspect of the present invention, there is provided a temperature-controllable cryoablation system, including a catheter, a fluid-conveying unit and a control unit, wherein the catheter includes a central cavity and a balloon located at the distal end of the catheter, and an input channel allowing a freezing fluid to be input into the balloon and an outflow channel allowing the freezing fluid to flow out of the balloon are provided in the central cavity; the fluid-conveying unit supplies the freezing fluid and discharges the freezing fluid; and the control unit controls the fluid-conveying unit so as to control, according to a target temperature value, the temperature of the balloon to be close to the target temperature value.

Preferably, the control unit includes a cascade PID control loop to control a temperature variation trend of the balloon, such that the temperature of the balloon is close to the target temperature value.

Preferably, the cascade PID control loop includes a main PID control loop (PID1) and an auxiliary PID control loop (PID2), the main PID control loop performs temperature variation trend control, and the auxiliary PID control loop performs pressure control.

Preferably, in the main PID control loop, the temperature serves as a target regulated variable, and in the auxiliary PID control loop, the pressure serves as an actual regulated variable; after the target temperature value is set, the main PID control loop and the auxiliary PID control loop perform linkage control to regulate the target regulated variable based on a change of the actual regulated variable, such that the temperature of the balloon is close to the target temperature value.

Preferably, the temperature of the balloon is infinitely close to the target temperature value.

Preferably, when the temperature of the balloon is close to the target temperature value, the lowest value of the temperature of the balloon is not less than the target temperature value.

Preferably, the target temperature value is a parameter prestored in the control unit and may be set by a human-machine interaction unit of the control unit.

Preferably, the main PID control loop regulates the temperature through pressure adjustment according to the target temperature value; the auxiliary PID control loop regulates the pressure through an opening degree of a flow regulating valve according to a pressure input by the main PID control loop.

Preferably, the main PID control loop and the auxiliary PID control loop calculate a regulated quantity and a control quantity according to the following equations respectively:

$$\text{control quantity}: u(t) = \Delta u(t) + \text{constant}$$

regulated quantity: $$\Delta u\ (t)\ = Kp\left[e(t) + \frac{1}{Ti}\int_0^t e(t)dt + Td\frac{de(t)}{dt}\right]$$

wherein e(t) is an error, and e(t)=target value-measured value;

Kp is a proportional gain;
Ti is an integration time; and
Td is differential action strength.

Preferably, an input side of the input channel of the catheter is connected to a fluid-conveying tube of the fluid-conveying unit, and an outflow side of the outflow channel of the catheter is connected with a fluid recovery tube of the fluid-conveying unit; an input-side pressure sensor and an input-side flow regulating valve are provided on the input side of the input channel of the catheter.

Preferably, the cryoablation system is further provided with at least one temperature sensor for detecting the temperature of the balloon.

According to another aspect of the present invention, there is provided a method for controlling a temperature of a balloon in a cryoablation system, including:
performing cascade PID control over the temperature of the balloon according to a set target temperature value, such that the temperature of the balloon is close to the target temperature value.

Preferably, the cascade PID control includes main PID control and auxiliary PID control, the main PID control is temperature variation trend control, and the auxiliary PID control is pressure control.

Preferably, in the main PID control, the temperature serves as a target regulated variable, and in the auxiliary PID control, the pressure serves as an actual regulated variable; after the target temperature value is set, the main PID control and the auxiliary PID control are linked to regulate the target regulated variable based on a change of the actual regulated variable, such that the temperature of the balloon is close to the target temperature value.

Preferably, the temperature of the balloon is infinitely close to the target temperature value.

Preferably, when the temperature of the balloon is close to the target temperature value, the lowest value of the temperature of the balloon is not less than the target temperature value.

Preferably, in the main PID control, the temperature is regulated through pressure adjustment according to the target temperature value; in the auxiliary PID control, the pressure is regulated through an opening degree of a flow regulating valve according to a pressure input by the main PID control.

Preferably, in the main PID control and the auxiliary PID control, a regulated quantity and a control quantity are calculated according to the following equations respectively:

control quantity:

$$u(t)=\Delta u(t)+constant$$

regulated quantity:

$$\Delta u\ (t)\ = Kp\left[e(t) + \frac{1}{Ti}\int_0^t e(t)dt + Td\frac{de(t)}{dt}\right]$$

wherein e(t) is an error, and e(t)=target value-measured value;

Kp is a proportional gain;
Ti is an integration time; and
Td is differential action strength.

## BRIEF DESCRIPTION OF DRAWINGS

The present invention will be described in more detail below with reference to embodiments and the accompanying drawings, in which:

FIG. 1 is a schematic structural diagram of a cryoablation system according to an embodiment of the present invention;
FIG. 2 shows a schematic diagram in which an input side and a discharge side of a catheter are connected with a fluid-conveying unit;

FIG. 3 shows a control principle diagram according to an embodiment of the present invention;

FIG. 4 is a schematic structural diagram of a catheter according to an embodiment of the present invention;

FIG. 5 shows a temperature graph according to an embodiment of the present invention; and

FIG. 6 shows a temperature variation graph according to an embodiment of the present invention.

## DESCRIPTION OF EMBODIMENTS

The technical solution of the present invention is further described in detail below by way of exemplary embodiments and with reference to the drawings, but the present invention is not limited to the following embodiments.

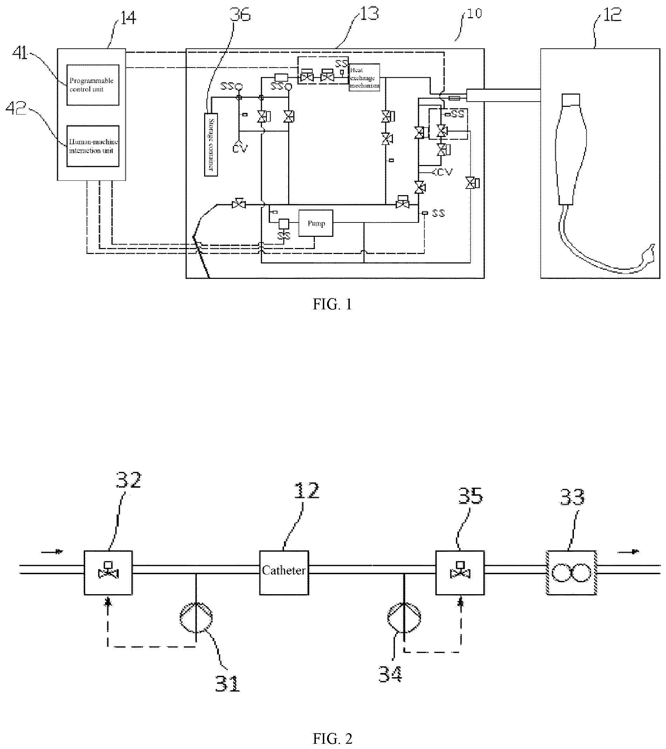

FIG. 1 shows a schematic structural diagram of a temperature-controllable cryoablation system 10 according to an embodiment of the present invention. As shown in FIG. 1, the system 10 may include a catheter 12, a fluid-conveying unit 13, and a control unit 14.

The catheter 12 includes an elongate main tube including a central cavity, and the main tube has a distal end provided with an expandable element, such as a balloon. The distal end generally refers to the end away from an operator and close to a patient, and a proximal end generally refers to the end close to the operator. One or more channels or conduits, such as fluid-conveying channels, fluid recovery channels, or the like, are provided in the central cavity of the main tube to provide fluid communication, mechanical communication and/or electrical communication between a proximal portion and a distal portion of the main tube. The proximal end of the fluid-conveying channel extends to the proximal end of the catheter, for example, is connected with a fluid-conveying tube of a freezing-fluid storage container of the fluid-conveying unit 13. The distal end of the fluid-conveying channel extends into the balloon, may be coiled or wrapped around a part of a shaft in a lumen of the balloon, or provided in the balloon in other ways, and allows a freezing fluid to be injected into the balloon through one or more holes. The distal end of the fluid recovery channel is communicated with the lumen of the balloon, the proximal end of the fluid recovery channel extends to the proximal end of the catheter through the main tube, and the gasified freezing fluid flows into a freezing-fluid recovery system through a fluid recovery tube of the fluid-conveying unit 13 connected with the fluid recovery channel.

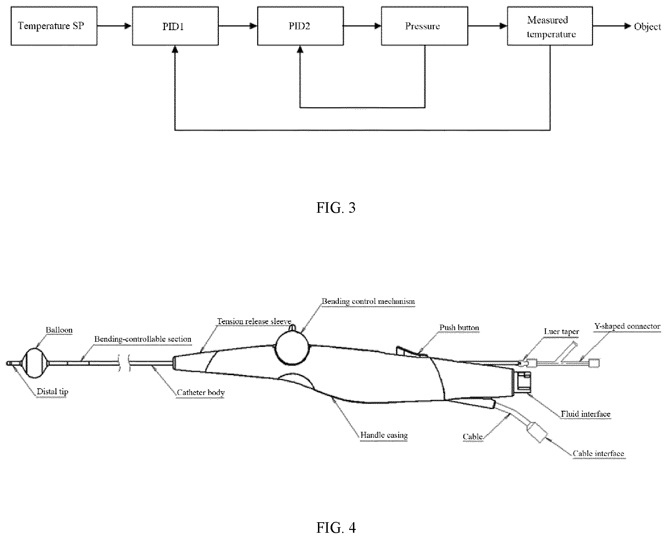

FIG. 4 shows an exemplary structure of the catheter according to an embodiment of the present invention; the catheter has a proximal end provided with an operating handle and various interfaces, and a distal end provided with a balloon, and one or more temperature sensors are provided in the balloon.

The fluid-conveying unit 13 includes a container and a pipeline which are configured to convey and discharge the freezing fluid, such as the freezing-fluid storage container 36, the freezing-fluid recovery system, or the like. The fluid-conveying unit 13 further includes a pump, a valve, a heat exchange mechanism, and control elements, such as a pressure sensor, a mass flow sensor, a temperature sensor, or the like, for data acquisition and flow regulation during delivery, recovery and/or recirculation of the freezing fluid conveyed into the balloon at the distal end of the catheter. The heat exchange mechanism may control a temperature of the freezing fluid prior to delivery of the freezing fluid to the balloon at the distal end of the catheter. Furthermore, the fluid-conveying unit 13 includes one or more check valves or pressure relief valves CV, and the check valve or the pressure relief valve leading to the atmosphere or the fluid recovery system may be automatically opened when a pressure level or flow in a part of the system exceeds a required or predetermined level. The valve and the control element in the fluid-conveying unit 13 may control the pressure of the freezing fluid.

The control unit 14 may include one or more controllers, processors, and/or software modules, for example, include a programmable control unit 41, a human-machine interaction (HMI) unit 42, or the like, in one embodiment. These controllers, processors and/or software modules include instructions or algorithms for controlling the fluid-conveying unit 13, which will be described in detail below.

According to an embodiment of the present invention, the programmable control unit 41 centrally processes and detects an input signal and outputs an instruction to an actuator of the fluid-conveying unit 13, to provide an automatic operation sequence or process and a target executing sequence or process. Using the HMI unit 42, the operator may provide an onsite instruction or a modified parameter, or the like, and the programmable control unit 41 receives the instruction and/or the parameter and sends the instruction to the actuator of the fluid-conveying unit 13 through a calculation.

FIG. 2 shows a schematic diagram in which an input side and a discharge side of the catheter 12 is connected to the fluid-conveying unit 13. As shown in FIG. 2, the input side of the catheter 12 is connected to the fluid-conveying tube of the fluid-conveying unit 13, and the discharge side of the catheter 12 is connected with the fluid recovery tube. The input side of the catheter refers to the part of the cryoablation system 10 before the delivery of the freezing fluid to the catheter, and the discharge side of the catheter refers to the part of the cryoablation system 10 after discharge of the freezing fluid from the catheter. An input-side pressure sensor 31, an input-side flow regulating valve 32, an input-side temperature sensor, or the like, may be provided on the input side of the catheter 12, and a discharge-side mass flow sensor 33, a discharge-side pressure sensor 34, a discharge-side flow regulating valve 35, a discharge-side temperature sensor, or the like, may be provided on the discharge side of the catheter 12. The pressure or flow of the freezing fluid is controlled by the fluid-conveying unit 13 and then conveyed into the balloon at the distal end of the catheter 12. The freezing fluid is discharged out of the catheter 12 through the fluid recovery tube on the discharge side after physical state transition.

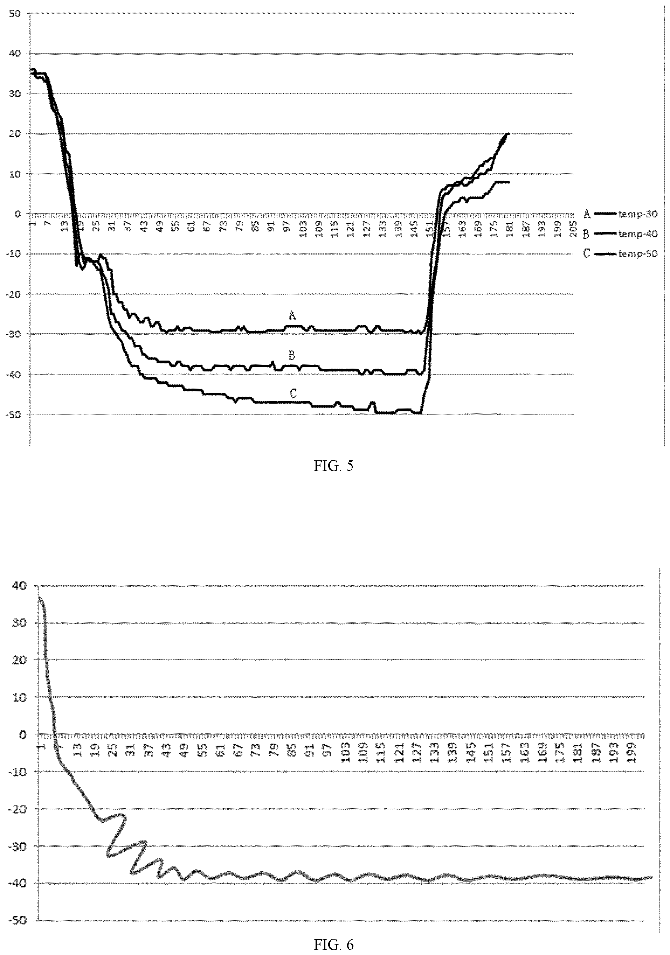

According to a specific embodiment of the present invention, as shown in FIG. 2, an input pressure of the freezing fluid may be fed back from a signal of the pressure sensor 31 on the input side of the catheter 12, and a discharge pressure of the freezing fluid may be fed back from a signal of the discharge-side pressure sensor 34 on the discharge side of the catheter 12. According to the present invention, the input pressure of the freezing fluid is required to be controlled, such that a temperature of the balloon at the distal end of the catheter 12 may be close to a target temperature value in a case where the freezing fluid at a certain flow is continuously supplied to the balloon of the cryoablation system (thereby providing a continuous amount of freezing for affected tissue). According to an embodiment of the present invention, the temperature of the balloon may be infinitely close to the target temperature value. When the temperature of the balloon is close to the target temperature value, the lowest value of the temperature of the balloon is not less than the target temperature value. For example, reference is made to the temperature variation curve of FIG. 6 according to an embodiment of the present invention.

In the present invention, the temperature of the balloon is close to the target temperature value, which includes not only a temperature variation trend that the temperature of the balloon gradually decreases and approaches the target temperature value, but also a temperature variation trend that the temperature of the balloon rapidly decreases and approaches the target temperature value. In some embodiments, a speed or time in which the temperature of the balloon approaches the target temperature value may be adjusted by setting control parameters. In the present invention, the temperature of the balloon fluctuates in the decreasing process, and therefore, the lowest value of the temperature of the balloon is the lowest value of the fluctuation curve.

A cascade PID control strategy may be adopted for control over the temperature of the balloon at the distal end of the catheter 12; specifically, a cascade PID control loop includes a main PID control loop PID1 and an auxiliary PID control loop PID2, the main PID control loop PID1 performs temperature variation trend control, and the auxiliary PID control loop PID2 performs pressure control. The control over the temperature variation trend in the present invention means that in the control process, the main PID control loop PID1 continuously obtains actual temperature values of the balloon by means of the sensor, and outputs corresponding pressure targets according to the actual temperature values, so as to regulate the pressure and the flow, thereby controlling the temperature variation trend. In the main PID control loop PID 1, the temperature serves as a target regulated variable, and in the auxiliary PID control loop PID2, the pressure serves as an actual regulated variable. After the required target temperature value is set, algorithms of the main PID control loop and the auxiliary PID control loop are linked to regulate the target regulated variable based on a change of the actual regulated variable, such that the temperature of the balloon is infinitely close to the target temperature value. The actual regulated variable has a relatively quick influence on a regulation action, and the change of the actual regulated variable may directly influence the target regulated variable. The target temperature value in the present invention is a parameter prestored in the control unit and may be set or regulated by the HMI unit of the control unit. An exemplary calculation method for realizing the controllable temperature of the balloon will be described in detail below. The controllable temperature in the present invention means that the variation trend of the temperature of the balloon may be controlled.

After a derivation according to the Bernoulli equation and a fluid continuity equation of hydromechanics, under a condition that a ratio of an outlet pressure to an inlet pressure of the regulating valve in the cryoablation system is greater than a critical pressure ratio, the following flow coefficient equation of the regulating valve may be obtained:

$$C_v = \frac{V}{16.05\sqrt{\frac{(P1^2 - P2^2)}{SG \cdot T}}} \quad (1)$$

wherein P1 is the inlet pressure of the regulating valve, i.e., a measured value of a regulating-valve upstream pressure sensor; P2 is the outlet pressure of the regulating valve, i.e., a measured value of a regulating-valve downstream pressure sensor; SG is specific gravity of a passing medium, T is a temperature of the passing medium, Cv is a flow coefficient of the regulating valve, and V is a volume flow (in SCFM).

Q is a flow of the freezing fluid, i.e., a measured value of a mass flow meter 33 (in SCCM), and therefore, a relationship between Q and V is:

$$Q = kV \quad (2)$$

wherein k is a constant and is a coefficient in a unit conversion process of Q and V

From the equations (1) and (2), the following equation is obtained:

$$Q = 16.05k C_v \sqrt{\frac{P1^2 - P2^2}{SG * T}} \quad (3)$$

The following relationship between an opening degree of the regulating valve and the flow is also known:

$$Q = \frac{Q_{max}}{R}\left[1 + (R-1)\frac{L'}{L}\right] \quad (4)$$

$$R = \frac{Q_{max}}{Q_{min}} \;;$$

wherein R is an adjustable ratio, and

L is a total stroke of the valve, L' is a certain flow stroke, i.e., the opening degree of the regulating valve, Qmin is a minimum control flow of the regulating valve, and Qmax is a maximum control flow of the regulating valve.

The following relationship between P2 and L' may be obtained from the equations (3) and (4):

$$P2 = \frac{\sqrt{(16.05kCvRL)^2 P1^2 - Qmax^2 S(}}{16.05kCvRL}$$

There also exists the following equation (see, for example, the detailed derivation and description of equations 4 and 5 in Chinese patent application No. CN107307901A (CN201710481942X), and this equation is slightly rewritten in form):

$$\left(\frac{T1\rho1}{T2\rho2} - 1\right)P = cQ^2 \quad (6)$$

wherein T1 is the in-balloon temperature, i.e., a measured value of an in-balloon temperature sensor; ρ1 is a density of gas in the balloon, P is a pressure value of the discharge side sensor 34, and T2 is the temperature of the freezing fluid in a pipeline near the valve 35; ρ2 is a density of the freezing fluid in the pipeline near the valve 35, Q is the measured value of the mass flow meter 33, and c is a coefficient, i.e., a calculated value of a parameter of a particular pipeline.

The following relationship between the regulating-valve downstream pressure P2 and the in-balloon temperature T1 may be obtained from the equations (3) and (6):

$$P2 = \sqrt{P_1^2 - \frac{\left(\frac{T_1\rho_1}{T_2\rho_2} - 1\right)P \cdot SG \cdot T}{16.05^2 k^2 Cv^2 c}} \quad (7)$$

The relationship between the opening degree L' of the regulating valve and the flow, the relationship between the opening degree L' of the regulating valve and the regulating-valve downstream pressure P2, and the relationship between the regulating-valve downstream pressure P2 and the in-balloon temperature T1 may be derived from the above equations, and values derived from the above-mentioned equations are theoretical values. Therefore, the opening degree L' of the regulating valve may be regulated using the cascade PID control strategy, thereby regulating the in-balloon temperature T1 to infinitely approach a target temperature.

The specific control strategy is illustrated with reference to FIG. 3.

In FIG. 3, each reference numeral has the following meaning:

temperature SP: set temperature value;
pressure: input-side regulated pressure, i.e., P2, measured value of input-side-regulating-valve downstream pressure sensor;
object: input-side regulating valve.

Regulated quantity:

$$\Delta u\ (t) = Kp\left[e(t) + \frac{1}{Ti}\int_0^t e(t)dt + Td\frac{de(t)}{dt}\right] \qquad (8)$$

wherein e(t) is an error, and e(t)=target value-measured value;

Kp is a proportional gain;
Ti is an integration time; and
Td is differential action strength.

Control quantity:

$$u(t)=\Delta u(t)+constant \qquad (9)$$

wherein the constant is an original initial value in the PID control, i.e., a value obtained according to accumulated test data under a specific condition.

Specifically, in the cryoablation system according to the present invention, during the control over the variation trend of the in-balloon temperature, the pressure of the freezing fluid is controlled by adjusting the opening degree of the input-side regulating valve.

The specific control process is exemplified as follows.

First, the target temperature value may be set in the cryoablation system according to the present invention. For example, the target temperature value may be set by a user using a user interface (not shown) or set in advance in the system. As shown in FIG. 3, the target temperature value may be supplied to the main PID control loop. A target pressure value at the set temperature may be calculated according to the equation (7) and the set target temperature. The main PID control loop (PID1) calculates an output signal (i.e., the pressure of the input-side regulating valve after adjustment) using the equations (8) and (9) of the PID control algorithm, and outputs the output signal to the auxiliary PID control loop (PID2). A D/A conversion may be involved in this process.

The auxiliary PID control loop receives a pressure regulation signal of the main PID control loop. According to the equation (5), a target value L' of the opening degree of the regulating valve may be calculated under a condition of knowing the regulating-valve downstream pressure P2. An actual control quantity of the opening degree of the regulating valve (the regulated opening degree of the regulating valve) is calculated using the equations (8) and (9) of the PID algorithm, and the actual control quantity of the opening degree of the regulating valve is outputted to an input-side regulating unit, such as the input-side flow regulating valve 32, or the like, so as to control a stroke of the actuator or a switching frequency of the regulating valve, thereby controlling the input pressure of the freezing fluid of the fluid-conveying unit 13.Ultimately, the temperature of the balloon at the distal end of the catheter 12 is controlled to infinitely approach the set target temperature value.

The auxiliary PID control loop obtains a theoretical value of the pressure to be controlled according to the equation 7, and then, parameters of the auxiliary PID control loop (PID2) are tuned to realize a basic temperature curve of the system. The basic temperature curve in the present invention refers to a temperature curve obtained under control of a single control loop without any feedback. Since constant input of a fixed quantity of freezing fluid by single PID control may cause the temperature of the balloon to continuously drop beyond the target temperature, the main PID control loop (PID 1) is added to regulate the pressure control signal received by the auxiliary PID control loop, thereby regulating the quantity of the freezing fluid. By tuning an output upper limit value, an output lower limit value, a proportional value, an integral value and a differential value of the control algorithm of the main PID control loop (PID1), a final signal value (i.e., a pressure control target) output to the auxiliary PID control loop allows the temperature value to infinitely approach the target temperature.

For example, the specific process of tuning the PID parameters (the target value, the measured value, the output upper limit value, the output lower limit value, a proportional parameter, an integral parameter, a differential parameter and a control period) may include: setting the target temperature value and the temperature measured value, limiting a range of a maximum value and a minimum value of an output calculation result of the main PID control loop, setting the proportional parameter, inputting the calculated output signal of the main PID control loop to the auxiliary PID control loop through, for example, an AD conversion, taking the signal received by the auxiliary PID control loop as the pressure control target value, and adjusting, by the auxiliary PID control loop, the opening degree of the valve to realize a target pressure according to the target value of the pressure. By analyzing the temperature curve, the proportional parameter is increased or decreased, and the system achieves a basic response speed and a deviation e(t). Then, the integral parameter is added to accumulate the deviation e(t), such that e(t) is eliminated completely, but frequent fluctuations of the system are increased. Finally, the differential parameter is added to prejudge a change condition of e(t), thus realizing advanced control and reducing a system adjustment time. Based on the above-mentioned process, the temperature of the balloon may be infinitely close to the target temperature value.

According to an embodiment of the present invention, the variation trend of the temperature of the balloon is controlled by controlling the input pressure of the freezing fluid, such that the temperature of the balloon is infinitely close to the set target temperature value. The temperature of the balloon refers to the measured temperature of the balloon. The set target temperature value may be adjusted as required using a HMI interface, such that different levels of temperature control (for example, a range from -50°C to -30°C, or other suitable temperature ranges) may be realized within a certain pressure range.

When the cryoablation system works stably, the temperature of the balloon at the distal end of the catheter 12 is in a certain range using the above-mentioned control method, and different levels of adjustment of the temperature may also be realized; that is, different target temperature values may be set as required. Thus, the operative risk is small, and a safer and more stable ablation mode is achieved when the same ablation depth is reached.

FIG. 5 shows a temperature graph according to an embodiment of the present invention. The horizontal axis represents time, the vertical axis represents balloon temperature, and after the pressure of the freezing fluid is controlled using the control method and the algorithm described above, the variation trend of the temperature in the balloon at the distal end of the catheter 12 may be controlled effectively, and the temperature of the balloon may infinitely approach the set target temperature value. For example, in the embodiment shown in FIG. 5, the target temperature value of the balloon may be controlled within the range from -50°C to -30°C.

FIG. 6 shows a schematic temperature variation graph according to an embodiment of the present invention, in which the horizontal axis represents time and the vertical axis represents balloon temperature. The curve is a temperature variation curve according to an embodiment of the present invention, and the target temperature of the balloon may be precisely controlled using the above-mentioned cascade PID control strategy, such that the temperature of the balloon is infinitely close to the target temperature.

The embodiments of the present invention are not limited to the above-mentioned embodiments, and various changes and modifications in form and detail may be made to the present invention by those skilled in the art without departing from the spirit and scope of the present invention, and these changes and modifications are considered to fall within the scope of the present invention.

## Claims

1. A temperature-controllable cryoablation system, comprising a catheter, a fluid-conveying unit and a control unit, wherein the catheter comprises a central cavity and a balloon located at the distal end of the catheter, and an input channel allowing a freezing fluid to be input into the balloon and an outflow channel allowing the freezing fluid to flow out of the balloon are provided in the central cavity; the fluid-conveying unit supplies the freezing fluid and discharges the freezing fluid; and the control unit controls the fluid-conveying unit so as to control, according to a target temperature value, the temperature of the balloon to be close to the target temperature value.

2. The temperature-controllable cryoablation system according to claim 1, wherein the control unit comprises a cascade PID control loop to control a temperature variation trend of the balloon, such that the temperature of the balloon is close to the target temperature value.

3. The temperature-controllable cryoablation system according to claim 2, wherein the cascade PID control loop comprises a main PID control loop (PID1) and an auxiliary PID control loop (PID2), the main PID control loop performs temperature variation trend control, and the auxiliary PID control loop performs pressure control.

4. The temperature-controllable cryoablation system according to claim 3, wherein in the main PID control loop, the temperature serves as a target regulated variable, and in the auxiliary PID control loop, the pressure serves as an actual regulated variable; after the target temperature value is set, the main PID control loop and the auxiliary PID control loop perform linkage control to regulate the target regulated variable based on a change of the actual regulated variable, such that the temperature of the balloon is close to the target temperature value.

5. The temperature-controllable cryoablation system according to any one of claims 1 to 4, wherein the temperature of the balloon is infinitely close to the target temperature value.

6. The temperature-controllable cryoablation system according to claim 5, wherein when the temperature of the balloon is close to the target temperature value, the lowest value of the temperature of the balloon is not less than the target temperature value.

7. The temperature-controllable cryoablation system according to any one of claims 1 to 4, wherein the target temperature value is a parameter prestored in the control unit and may be set by a human-machine interaction unit of the control unit.

8. The cryoablation system according to claim 3 or 4, wherein the main PID control loop regulates the temperature through pressure adjustment according to the target temperature value; the auxiliary PID control loop regulates the pressure through an opening degree of a flow regulating valve according to a pressure input by the main PID control loop.

9. The cryoablation system according to any one of claims 1 to 4, wherein the main PID control loop and the auxiliary PID control loop calculate a regulated quantity and a control quantity according to the following equations respectively:

control quantity:

$$u(t) = \Delta u(t) + \text{constant}$$

regulated quantity:

$$\Delta u\ (t) = Kp\left[e(t) + \frac{1}{Ti}\int_0^t e(t)dt + Td\frac{de(t)}{dt}\right]$$

wherein e(t) is an error, and e(t)=target value-measured value;

      Kp is a proportional gain;
      Ti is an integration time; and
      Td is differential action strength.

10. The cryoablation system according to any one of claims 1 to 4, wherein an input side of the input channel of the catheter is connected with a fluid-conveying tube of the fluid-conveying unit, and an outflow side of the outflow channel of the catheter is connected with a fluid recovery tube of the fluid-conveying unit; an input-side pressure sensor and an input-side flow regulating valve are provided on the input side of the input channel of the catheter.

11. The cryoablation system according to any one of claims 1 to 4, wherein the cryoablation system is further provided with at least one temperature sensor for detecting the temperature of the balloon.

12. A method for controlling a temperature of a balloon in a cryoablation system, comprising: performing cascade PID control over the temperature of the balloon according to a set target temperature value, such that the temperature of the balloon is close to the target temperature value.

13. The method according to claim 12, wherein the cascade PID control comprises main PID control and auxiliary PID control, the main PID control is temperature variation trend control, and the auxiliary PID control is pressure control.

14. The method according to claim 13, wherein in the main PID control, the temperature serves as a target regulated variable, and in the auxiliary PID control, the pressure serves as an actual regulated variable; after the target temperature value is set, the main PID control and the auxiliary PID control are linked to regulate the target regulated variable based on a change of the actual regulated variable, such that the temperature of the balloon is close to the target temperature value.

15. The method according to any one of claims 12 to 14, wherein the temperature of the balloon is infinitely close to the target temperature value.

16. The method according to claim 15, wherein when the temperature of the balloon is close to the target temperature value, the lowest value of the temperature of the balloon is not less than the target temperature value.

17. The method according to claim 13 or 14, wherein in the main PID control, the temperature is regulated through pressure adjustment according to the target temperature value; in the auxiliary PID control, the pressure is regulated through an opening degree of a flow regulating valve according to a pressure input by the main PID control.

18. The method according to any one of claims 12 to 14, wherein in the main PID control and the auxiliary PID control, a regulated quantity and a control quantity are calculated according to the following equations respectively:

control quantity:

$$u(t)= \triangle u(t)+constant$$

regulated quantity:

$$\Delta u\ (t)\ =Kp\left[e(t)+\frac{1}{Ti}\int_0^t e(t)dt+Td\frac{de(t)}{dt}\right]$$

wherein e(t) is an error, and e(t)=target value-measured value;

Kp is a proportional gain;
Ti is an integration time; and
Td is differential action strength.

FIG. 1

FIG. 2

| Temperature SP | → | PID1 | → | PID2 | → | Pressure | → | Measured temperature | → Object |

FIG. 3

Balloon

Bending control mechanism

Tension release sleeve

Push button

Luer taper

Y-shaped connector

Bending-controllable section

Distal tip

Catheter body

Handle casing

Cable

Fluid interface

Cable interface

FIG. 4

FIG. 5

FIG. 6

## INTERNATIONAL SEARCH REPORT

| International application No. |
| --- |
| **PCT/CN2020/107401** |

| A. CLASSIFICATION OF SUBJECT MATTER |
| --- |
| A61B 18/02(2006.01)i |
| According to International Patent Classification (IPC) or to both national classification and IPC |

| B. FIELDS SEARCHED |
| --- |
| Minimum documentation searched (classification system followed by classification symbols) |
| A61B |
| Documentation searched other than minimum documentation to the extent that such documents are included in the fields searched |
| Electronic data base consulted during the international search (name of data base and, where practicable, search terms used) |
| CNABS, CNTXT, CNKI: 球囊, 温度, 调, 控, 冷冻, 冷疗, 消融, 压力, 串级, 主, 副, PID, 流量, 阀; GBTXT; CATXT; EPTXT; USTXT; VEN; WOTXT: balloon, temperature, control, monitor, adjust, ablate, cool, cryoablation, cascade, primary, main, slave, sub, vice, pressure, flow, valve |

| C. DOCUMENTS CONSIDERED TO BE RELEVANT | | |
| --- | --- | --- |
| Category* | Citation of document, with indication, where appropriate, of the relevant passages | Relevant to claim No. |
| PX | CN 110464444 A (SYNAPTIC MEDICAL TECHNOLOGY (BEIJING) CO., LTD.) 19 November 2019 (2019-11-19) <br> claims 1-18 | 1-18, |
| X | CN 109498145 A (KOSSEL MEDTECH (SUZHOU) CO., LTD.) 22 March 2019 (2019-03-22) <br> description, paragraphs 24-37, and figures 1-4 | 1, 5, 6, 10, 11 |
| Y | CN 109498145 A (KOSSEL MEDTECH (SUZHOU) CO., LTD.) 22 March 2019 (2019-03-22) <br> description, paragraphs 24-37, and figures 1-4 | 2-4, 7-9, 12-18 |
| Y | CN 104792079 A (LIUZHOU VOCATIONAL & TECHNICAL COLLEGE) 22 July 2015 (2015-07-22) <br> description, paragraphs [0011]-[0018], and figure 1 | 2-4, 7-9, 12-18 |
| X | US 2014276698 A1 (MEDTRONIC CRYOCATH LP) 18 September 2014 (2014-09-18) <br> description, paragraphs 18-31, and figures 1-3 | 1, 5, 6, 10, 11 |
| Y | US 2014276698 A1 (MEDTRONIC CRYOCATH LP) 18 September 2014 (2014-09-18) <br> description, paragraphs 18-31, and figures 1-3 | 2-4, 7-9, 12-18 |

☑ Further documents are listed in the continuation of Box C.   ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **14 October 2020** | **05 November 2020** |

| Name and mailing address of the ISA/CN | Authorized officer |
| --- | --- |
| **China National Intellectual Property Administration (ISA/ CN)** <br> **No. 6, Xitucheng Road, Jimenqiao Haidian District, Beijing 100088** <br> **China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2020/107401**

**C.      DOCUMENTS CONSIDERED TO BE RELEVANT**

| Category* | Citation of document, with indication, where appropriate, of the relevant passages | Relevant to claim No. |
|---|---|---|
| Y | CN 206019334 U (SINOSTEEL EQUIPMENT & ENGINEERING CO., LTD.) 15 March 2017 (2017-03-15) <br> description, paragraphs 48-83, and figures 1-2 | 2-4, 7-9, 12-18 |
| A | CN 109662775 A (SHENZHEN HUAHAN BIOTECHNOLOGY CO., LTD.) 23 April 2019 (2019-04-23) <br> entire document | 1-18, |

Form PCT/ISA/210 (second sheet) (January 2015)

**INTERNATIONAL SEARCH REPORT**
Information on patent family members

International application No.

**PCT/CN2020/107401**

| Patent document cited in search report | | | Publication date (day/month/year) | Patent family member(s) | | | Publication date (day/month/year) |
|---|---|---|---|---|---|---|---|
| CN | 110464444 | A | 19 November 2019 | None | | | |
| CN | 109498145 | A | 22 March 2019 | None | | | |
| CN | 104792079 | A | 22 July 2015 | CN | 104792079 | B | 08 March 2017 |
| US | 2014276698 | A1 | 18 September 2014 | WO | 2014138866 | A1 | 18 September 2014 |
| | | | | US | 9750555 | B2 | 05 September 2017 |
| | | | | US | 2015223860 | A1 | 13 August 2015 |
| CN | 206019334 | U | 15 March 2017 | None | | | |
| CN | 109662775 | A | 23 April 2019 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 107307901 A

- CN 201710481942X